**Europäisches Patentamt**

**European Patent Office**

**Office européen des brevets**

(19)

(11) Publication number : **0 091 412 B2**

(12) **NEW EUROPEAN PATENT SPECIFICATION**

(45) Date of publication of the new patent specification : **11.03.92 Bulletin 92/11**

(51) Int. Cl.⁵ : **A61F 13/15**

(21) Application number : **83850068.4**

(22) Date of filing : **17.03.83**

(54) **Absorbent product.**

(30) Priority : **01.04.82 SE 8202090**

(43) Date of publication of application : **12.10.83 Bulletin 83/41**

(45) Publication of the grant of the patent : **06.05.87 Bulletin 87/19**

(45) Mention of the opposition decision : **11.03.92 Bulletin 92/11**

(84) Designated Contracting States : **AT BE CH DE FR GB IT LI LU NL**

(56) References cited :
EP-A- 0 027 303
AT-B- 292 917
AU-A-68 723 /81
DE-A- 2 854 792

(56) References cited :
US-A- 2 745 405
US-A- 3 860 003
US-A- 3 906 952
US-A- 4 253 461
US-A- 4 259 958
US-A- 4 326 528
US-A- 4 333 782

(73) Proprietor : **Mölnlycke AB**
**S-405 03 Göteborg (SE)**

(72) Inventor : **Widlund, Leif Urban Roland**
**Päronvägen 5**
**S-435 00 Mölnlycke (SE)**
Inventor : **Nedestam, Stig Staffan**
**Radavägen 80**
**S-435 00 Mölnlycke (SE)**

(74) Representative : **Alfredson, Stig et al**
**H. ALBIHNS PATENTBYRA AB Box 3137**
**S-103 62 Stockholm (SE)**

EP 0 091 412 B2

## Description

The present invention relates to a sanitary napkin, comprising an absorbent body and a jacket enclosing said body, said jacket comprising a fluid-permeable layer for being located close to the user's skin when in use, and a fluid-impermeable layer disposed on the opposed side, the back, of the absorbent body. At least the fluid-impermeable outer layer extends outside the two longitudinal lateral edges of the absorbent body so as to form two side margins each including an elastic means in the form of elastic string or the like, which are disposed substantially in the longitudinal direction of the napkin, each of said elastic means extending at least in the central portion of the napkin and being secured to the respective side margins in a pre-stretched condition.

Leakage to the side has been a problem when using sanitary napkins. This has been a problem in all types of sanitary napkins, both the older types, i.e. thick and wide napkins and in more modern types, e.g. so-called body-shaped napkins, which vary in thickness along their lengths and are relatively narrow in the midsection where they are thickest.

Sanitary napkins of the older uniformly thick types are often greatly deformed when used quite simply because they are too large and not fitted to the shape of the human body. Usually they are pressed together in the middle and bent along a longitudinal axis so that the front, the side in contact with the user, becomes convex with portions of the fluid-permeable layer facing out towards the sides at the same time as the fluid-impermeable back layer is more or less folded up resulting in a reduction in effective fluid-stopping surface. This means that fluid from the napkins can leak out through the folded out fluid-permeable front layer on both sides of the folded up fluid-impermeable back layer.

Attempts have been made to solve this problem by making the modern sanitary napkins so that when used they more closely follow the shape of the body and have their greatest absorption capacity where the need is greatest, i.e. by making them with relatively thick midsections, which are at the same time relatively narrow across. By virtue of this shape, the modern sanitary napkins are not deformed especially much during use, thus reducing edge leakage over the older types of board uniformly thick napkins. Despite this, however, side leakage is still a substantial problem even in body-shaped napkins. On occasions when fluid discharge is great, experience has shown that not all the fluid has time to be absorbed; rather a portion can still leak out to the sides and over the longitudinal edges of the napkin.

Developments of the last few years have, however, made the napkins thinner and thinner. This has been made possible both by compressing the absorbent material, and by using high-absorbent material.

Examples of such very thin and comfortable napkins are the so-called panty protectors, which are intended to be used in combination with tampons or instead of sanitary napkins of the older thicker types.

For such thin napkins, side leakage is of course an even greater problem than in the body-shaped napkins. Since they are usually very thin, they must be relatively broad even in the middle, making them liable to be greatly deformed when used, unfortunately often resulting in edge leakage in this type of napkins as well.

Many different attempts have been made to eliminate the occurrence of lateral leakage. One example is in thin napkins and panty protectors to arrange a number of longitudinal compressed areas in the absorbent body for the purpose of rapidly spreading the fluid longitudinally. These compressed areas are, however, far from sufficient to satisfactorily eliminate edge leakage.

It is also common to extend the fluid-impermeable back layer laterally and to fold it towards the side edges of the napkin. It is true that this prevents to a high degree liquid from the interior of the absorbent body from leaking out laterally, but fluid can of course still run out over the side in contact with the user, the front side, and thus on the outside of the fluid-impermeable layer folded up about the sides of the napkin.

It is also known to further widen the fluid-impermeable back layer so as to cover, in addition to the back and side edges of the napkin, also a portion of the front side in contact with the user during use. In such a design, the fluid already collected in the absorbent body is to be sure effectively enclosed, but instead there is the substantially disadvantage that the fluid-impermeable back layer folded in over the front side can cover a major portion of the napkin side facing the user when the napkin is deformed when used, whereby menstrual fluid can run directly out of the napkin on top of the portions of the back layer folded in over the front.

From such absorbent products as disposable diapers it is known to eliminate side leakage also by having the central portion of each side margin gathered to provide improved fit about the baby's thighs. In the panty-like diaper disclosed in US-A-4 333 782 the absorbent body is, for that purpose, enclosed in a jacket comprising a fluid-permeable layer for being located close to the user's skin when in use, and a fluid-impermeable layer disposed on the opposed side, the back, of the absorbent body. The two layers extend outside of the lateral edges of the absorbent body and longitudinally thereto, so as to form two side margins each including a substantially elongate elastic means. The elastic means extend substantially in the longitudinal direction of the product along at least the central portion thereof and are secured to said side margins in a prestretched condition.

This applies also to the panty-like diaper disclosed in AU-A-68 723/81, in which diaper the elastic means is arranged not only to improve the fit of the diaper around the leg of the user but also to create a pocket to contain the liquid. For the latter purpose the absorption body of the diaper is provided with flex regions and is rigid not only in its lower profile but also in its sides between the flex regions and the leg endireling elastic. This results in an unusually bulky undergarment-like diaper, which pocket-like bottom part it is not possible to introdude in samitary napkins.

Further, to have the central portion of each side margin of a sanitary napkin gathered like side margins on disposable diapers in order to provide improved fit about the user's thighs is, however, not possible either, since sanitary napkins are relatively small and strictly concentrated to their place of use, and disposable diapers are more or less like under-garments always covering a substantial part of the baby's body.

Despite the fact that attemps have been made previously to eliminate side leakage from such absorbent products as sanitary napkins, up to now, as revealed above, this problem has not been satisfactorily solved. Therefore the purpose of the present invention is to suggest a new improved sanitary napkin, in which the possibilities of lateral leakage are reduced to such a degree that this leakage problem is at least substantially eliminated.

For this purpose a sanitary napkin is suggested wherein the elastic means secured to the side margins which extend outside the two longitudinal lateral edges of the absorbent body are disposed closer to the external edges of said margins than to the external edges of the absorbent body, so as to cause the side margins to be raised against the border of the absorbent body in the direction towards the fluid-permeable layer and at least the midportion of the side margins to be contracted and held by the elastic means in their raised position whereby the margins serve as barriers against lateral leakage of fluid.

The outer layer portions extending beyond the side edges of the absorbent body create side flaps, and with their elastic means, the fluid-impermeable back layer forms a bowl surrounding the absorbent body in a sanitary napkin made according to the invention. The side flaps serving as a barrier against fluid leakage to the sides are located, when the product is used, always spaced from the side edge of the absorbent body, so that any fluid which runs over the front of the napkin and follows the side edges of the absorbent body, is collected by the barrier. Fluid which is not immediately absorbed by the absorbent body is this collected in the pockets formed by the side flaps on either side of the absorbent body and is gradually absorbed by the absorbent body.

In contrast to the case in the known sanitary napkins described here with portions of the fluid-impermeable back layer disposed about the side edges of the napkin the leakage barriers formed by the side flaps in a product made according to the invention are always disposed spaced both from the top side of the absorbent body and from its side edges. This is the case even when the sanitary napkin is compressed during use or otherwise deformed. The side flaps are folded by the elastic means, and therefore there is no question of sealing contact against the side edges of the absorbent body.

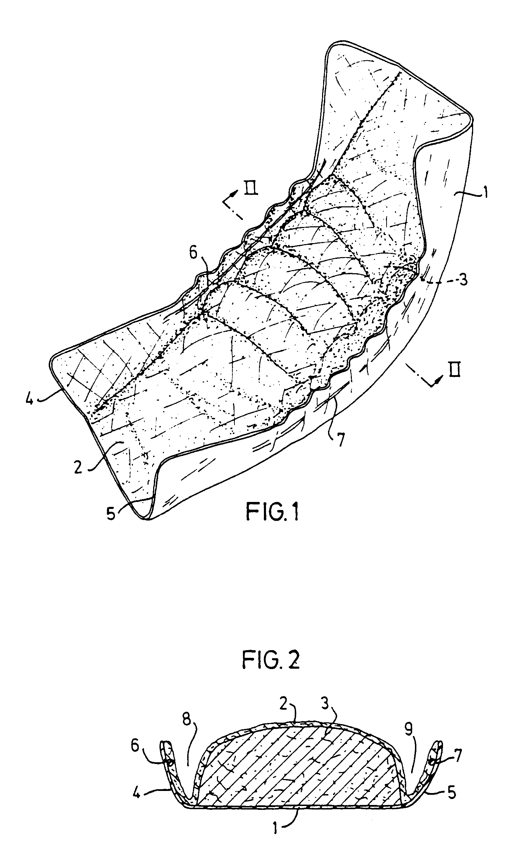

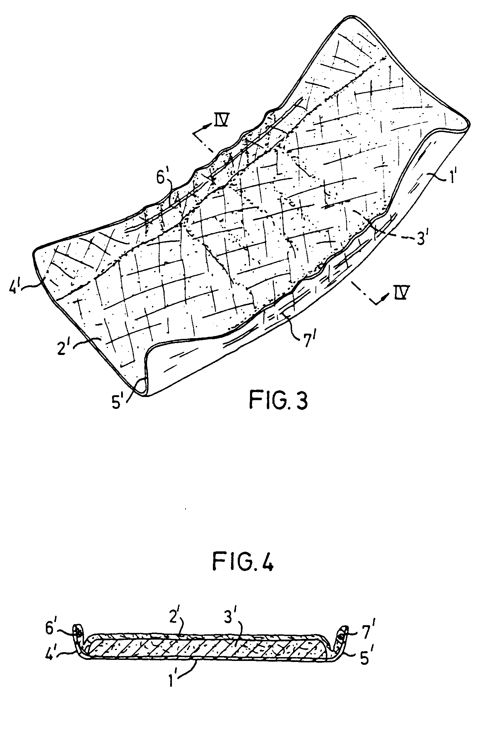

The invention will be described in more detail below with reference to two examples shown in the accompanying drawings. Fig. 1 shows a perspective view of a first embodiment of a sanitary napkin made according to the invention, which is relatively thick. Fig. 2 shows a cross section along the line II-II in Fig. 1. Fig. 3 shows a second embodiment of a thin sanitary napkin according to the invention. Finally, Fig. 4 shows a cross section along the line IV-IV in Fig. 3.

As can be seen from Figs. 1 and 2 a sanitary napkin made according to the invention comprises a fluid-impermeable layer 1, a fluid-permeable front layer 2, and an absorbent body 3 between these two outer layers. The two outer layers or jacket layers extend about the absorbent body and somewhat outside the same and are joined to each other there. The side flaps formed thereby have been labelled 4 and 5 in the drawing. Between the outer layers 1, 2 there are elastic threads 6, 7 mounted on the side flaps 4, 5 and extending spaced from the side edges of the absorbent body essentially longitudinally to the napkin. As can be seen from Fig. 1, the elastic means is only effective within the midportion of the napkin, where the side portions or flaps 4, 5 have been contracted and raised towards the fluid-permeable front layer 2. Fluid pockets 8, 9 are thus formed on both sides of the absorbent body 3, as can be clearly seen in Fig. 2.

Fig. 3 and 4 show a thin sanitary napkin or a so-called panty protector made according to the invention. It comprises a fluid-impermeable back layer 1', a fluid-permeable front layer 2' and an absorbent body 3' therebetween, which consists of very compressed cellulose material. The two outer layers 1', 2' are extended somewhat outside the absorbent body and are joined there to each other. Along the side flaps 4', 5' formed thereby, elastic threads 6', 7' are disposed between the two outer layers and spaced from the absorbent body. These threads are mounted when stretched and are active in the midportion of the napkin. In this case no real pockets are formed on either side of the thin absorbent body 3', but the raised side flaps 4', 5' form instead, as can be seen in Fig. 4, a sort of coaming on either side of the absorbent body, which prevents fluid from leaking laterally from the napkin.

The embodiments described above are suitably manufactured as follows.

A web of fluid-impermeable material is fed past a glue roller, which applies glue only to portions of this material intended therefor. Elastic threads are then applied to the fluid-impermeable web in the longitudinal direction of the web, and they are held stretched during the manufacture of the napkins.

Absorbent cores to the napkins are formed and fed with equal spacing on a web of fluid-permeable material. The web consisting of the fluid-impermeable material with the applied elastic threads is then applied over the fluid-permeable web and the absorbent cores, whereafter the composite passes through a pinching roller.

The two webs, i.e. the fluid-impermeable and fluid-permeable, and the elastic threads are bonded together by the pinching rollers, the bond being made by means of the previously applied adhesive, which is suitably hot melt. After the pinch rollers, the coherent web is then fed to a station where it is cut off to form the individual napkins.

As was mentioned in the preceding, adhesive can be applied by a roller to suitable portions of the fluid-impermeable layer, so that for example adhesive on longitudinal layer portions, where the elastic threads are subsequently to be disposed, is only applied within limited areas which are to be the midportions of the respective napkins. The elastic threads will thereby only provide elastic effect within the midportion of the finished napkin.

The method of manufacture described above is particularly simple. Thanks to the fact that the two outer layers or jacket layers extend outside the absorbent body around the same, the jacket can be sealed without the need of folding the layers. The absorbent cores can also be preshaped to a somewhat arched shape so that the side portions of the outer layers extending outside the core will be bent towards the fluid-permeable layer when the individual napkins are cut and the elastic means contracts. The elastic means causes said side portions of the outer layers to be raised to form the leakage barrier, thus preventing lateral leakage from both the interior of the absorbent body and from the front of the napkin. The manufacture of previously known napkins have necessitated a more or less complicated folding of the fluid-impermeable layer up or around the absorbent body to form a barrier, which only protects against leakage from the interior of the absorbent body.

The present invention is not limited to the embodiments described above, since a number of modifications are possible within the scope of the invention. For example, the elastic means do not need to be fixed in the middle of the napkin but can be joined to the outer layers along the entire length of the napkin. The invention is also applicable to all types of sanitary napkins and panty protectors regardless of their shape.

The absorbent cores need not be preshaped to an arched shape, but can be made in a number of different ways. In certain cases it can however be necessary to have folding means to fold up the side flaps extending beyond the absorbent body prior to cutting off the coherent web of napkins, so that the leakage barrier will be on the correct side of the napkin when the elastic means contracts.

A sanitary napkin according to the invention can also be made so that the fluid-permeable layer also encloses the side edges of the absorbent body and at least partially the side of the absorbent body facing away from the user when used. In this embodiment, only the fluid-impermeable layer extends laterally outside the absorbent body and forms there together with the elastic means the leakage barriers.

The two outer layers can of course also be joined to each other on either side edge of the absorbent body with portions of the fluid-impermeable outer layer extending farther out laterally than the permeable layer so that the fluid barriers are formed only by these fluid-impermeable outer portions.

The elastic means do not need to be securely fixed to any of the jacket layers over the entire midsection of the napkin, for example. Elastic threads or the like can be applied in special channels made by folding over the fluid-impermeable back layer and then need only be anchored at the ends, thus better exploiting the elastic means than if the threads were joined to the outer layer along its entire length.

Instead of elastic threads, bands or the like, the elastic means can also be special hot melt which is applied without pretensioning on either jacket layer. The molecular chains of the adhesive are changed in such a way when heated that they become elastically tensioned and thereby achieve the required contraction and raising of the side flaps of the napkin.

## Claims

1. Sanitary napkin, comprising an absorbent body (3) and a jacket enclosing said body, said jacket comprising a fluid-permeable layer (2) for being located close to the user's skin when in use, and a fluid-impermeable layer (1) disposed on the opposed side, the back, of the absorbent body, at least the fluid-impermeable outer layer (1) extending out-side the two longitudinal lateral edges of the absorbent body (3) so as to form two side margins (4, 5) each including an elastic means (6, 7) in the form of elastic strings or the like which are disposed substantially in the longitudinal direction of the napkin, each of said elastic means (6, 7) extending at least in the central portion of the napkin and being secured to the respective side margins in a pre-stretched condition, wherein the elastic means (6, 7) are disposed closer to the external edges of said margins than to the external edges of the absorbent body, so as to cause the side margins to the raised against the border of the absorbent body in the direction towards the fluid-permeable layer and so as to cause at least the midportion of the side margins (4, 5) to be contracted and held by the elastic means in their raised positions, whereby the side margins serve as barriers against lateral leakage of fluid.

2. Sanitary napkin according to Claim 1, wherein the two jacket layers (1, 2) extend outside the lateral edges of the absorbent body (3) and are joined to each other there.

3. Sanitary napkin according to Claim 1 or 2, wherein the elastic means (6, 7) consists of hot melt adhesive, which becomes permanently elastic only after heating.

**Patentansprüche**

1. Monatsbinde, umfassend einen absorbierenden Körper (3) und eine den Körper umgebende Umhüllung, welche Umhüllung eine beim Gebrauch näher dem Benützer befindliche fluiddurchlässige Schicht (2) und eine an der entgegengesetzten Seite, der Rückseite des absorbierenden Körpers befindliche fluidundurchlässige Schicht (1) umfaßt, wobei zumindest die fluidundurchlässige Außenschicht (1) über die beiden Längsseitenkanten des absorbierenden Körpers (3) unter Ausbildung von zwei Seitenrändern (4,5) vorsteht, von denen jeder ein elastisches Mittel (6,7) in Form elastischer Schnüre od.dgl. einschließt, die im wesentlichvn in Längsrichtung der Binde angeordnet sind, wobei sich jedes dieser elastischen Mittel (6,7) zumindest im Mittelabschnitt der Binde erstreckt und an dem jeweiligen Seitenrand in einem vorgespannten Zustand befestigt ist, in welcher Monatsbinde die elastischen Mittel (6,7) näher zu den Außenkanten dieser Ränder als zu den Außenkanten des absorbierenden Körpers angeordnet sind, um die Seitenränder gegen den Rand des absorbierenden Körpers in Richtung auf die fluiddurchlässige Schicht hin aufzurichten und zumindest den Mittelabschnitt der Seitenränder (4,5) zusammenzuziehen und von den elastischen Mitteln in ihren aufgerichteten Stellungen gehalten zu werden, wodurch die Seitenränder als Barriere gegen eine seitliche Fluidleckage dienen.

2. Monatsbinde nach Anspruch 1, worin die beiden Umhüllungsschichten (1,2) über die Seitenkanten des absorbierenden Körpers (3) vorstehen und dort miteinander verbunden sind.

3. Monatsbinde nach Anspruch 1 oder 2, worin die elastischen Mittel (6,7) aus einem Heißschmelzkleber bestehen, welcher erst nach Erwärmen permanent elastisch wird.

**Revendications**

1. Serviette hygiénique qui comprend une masse absorbante (3) et une enveloppe enfermant cette masse, ladite enveloppe comprenant une couche perméable aux fluides (2) qui vient se placer tout près de l'utilisatrice pendant l'utilisation, et une couche imperméable aux fluides (1) disposée de l'autre côté, au dos de la masse absorbante, au moins la couche extérieure imperméable aux fluides (1) s'étendant légèrement à l'extérieur des deux bords latéraux longitudinaux de la masse absorbante (3) de façon à former deux bordures latérales (4, 5), chacune d'elles comportant un moyen élastique allongé (6, 7) en forme de cordes élastiques ou d'éléments analogues qui sont disposés sensiblement dans le sens de l'axe longitudinal de la serviette, chacun desdits moyens élastiques (6, 7) s'étendant au moins dans la partie centrale de la serviette et étant fixé sur les bords latéraux respectifs en étant préétiré, les moyens élastiques (6, 7) de la serviette hygiénique étant disposés plus près des bords externes desdites bordures que des bords externes du corps absorbant, de façon à provoquer le soulèvement desdites bordures sur la lisière du corps absorbant en direction de la couche perméable aux fluides et de façon à provoquer la contraction d'au moins la partie centrale des bordures latérales (4, 5) et leur retenue par le moyen élastique dans leurs positions élevées, il s'ensuit que les bordures latérales servent de barrières contre les fuites latérales du fluide.

2. Serviette hygiénique selon la revendication 1, dont les deux couches (1, 2) de l'enveloppent s'étendent à l'extérieur, au-delà des bords latéraux de la masse absorbante (3) et sont reliées l'une à l'autre.

3. Serviette hygiénique selon la revendication 1 ou 2, dont les moyens élastiques (6, 7) sont constitués part des fils élastiques ou autres qui sont montés sous tension.

FIG. 1

FIG. 2

FIG. 3

FIG. 4